# EUROPÄISCHE PATENTSCHRIFT

(11) **EP 0 754 664 B1**
(45) Veröffentlichungstag und Bekanntmachung des Hinweises auf die Patenterteilung: **17.11.1999**
(21) Anmeldenummer: 96111235.6
(22) Anmeldetag: 12.07.1996
(51) Int. Cl.: C07C 5/09

(54) **Verfahren zur Herstellung von Alkenen durch partielle Hydrierung von Alkinen an Festbett-Palladium-Katalysatoren**
Process for preparing alkenes by partial hydrogenation of alkines on solid-bed-palladium-catalysts
Procédé pour la préparation d'alkènes par hydrogénation partielle d'alkynes sur des catalyseurs à lit fixe contenant du palladium

(30) Priorität: 20.07.1995 DE 19526473
(43) Veröffentlichungstag der Anmeldung: 22.01.1997
(73) Patentinhaber: BASF AKTIENGESELLSCHAFT, 67056 Ludwigshafen (DE)
(72) Erfinder: Bröcker, Franz Josef, Dr., 67061 Ludwigshafen (DE); Stroezel, Manfred, 68549 Ilvesheim (DE); Rheude, Udo, Dr., 67166 Otterstadt (DE)

(56) Entgegenhaltungen:
- EP-A- 0 412 415
- DE-A- 3 143 647
- US-A- 4 570 025
- DATABASE WPI Section Ch, Week 8408 Derwent Publications Ltd., London, GB; Class A41, AN 84-045214 XP002028642 & JP 59 005 127 A (SUMITOMO CHEMICAL KK) , 12.Januar 1984
- PATENT ABSTRACTS OF JAPAN vol. 007, no. 043 (C-152), 19.Februar 1983 & JP 57 193419 A (SHOWA DENKO KK), 27.November 1982,

## Beschreibung

Verfahren zur Herstellung von Alkenen durch partielle Hydrierung von Alkinen an Festbett-Palladium-Katalysatoren.

Die Erfindung betrifft ein technisch sehr vorteilhaftes Verfahren zur Herstellung von Alkenen, insbesondere von monosubstituierten Alkenen, durch Partialhydrierung der entsprechenden Alkine in flüssiger Phase an Palladium-Festbett-Trägerkatalysatoren unter Zusatz von Kohlenmonoxid (CO) zum Hydrierwasserstoff.

Die Hydrierung von Alkinen zu Alkenen ist Gegenstand eines umfangreichen Standes der Technik.

So wird in GB-A 871 804 eine verbesserte partielle Hydrierung von Acetylenverbindungen in Suspensionsfahrweise mit einem Pd-Katalysator, der mit Salzlösungen der Metalle Zn, Cd, Hg, Ga, In oder Tl behandelt wurde, beschrieben.

Ferner wird in der DE-A 24 31 929 ein Verfahren zur Herstellung von 2-Buten-1,4-diol durch Hydrieren von Butindiol in wäßriger Lösung an einem Katalysator, der Pd und eines der Elemente Zn oder Cd und wenigstens eines der Elemente Bi oder Te enthält, beschrieben. Als Katalysatorträger wird Bimsstein oder Aluminiumoxid verwendet.

Für die partielle Hydrierung der Dreifachbindung in Vitamin- und Riechstoffvorprodukten werden üblicherweise und, wie z.B in US-A 2 681 938 beschrieben, Blei-dotierte Pd-Katalysatoren, sogenannte Lindlar-Katalysatoren eingesetzt. Vielfach werden diese noch mittels Schwefelverbindungen desaktiviert, um die Selektivität zu steigern (JP-A 120 657/81).

Aus DE-A 26 19 660 ist schließlich ein Verfahren zur Herstellung von Butendiol bekannt, bei dem Butindiol in einem inerten Lösungsmittel in Anwesenheit eines Katalysators, der mit Kohlenmonoxid behandeltes metallisches Pd enthält, hydriert wird. Dieses Verfahren kann zusätzlich in Gegenwart von etwa 200 bis 2000 ppm CO im Hydrierwasserstoff durchgeführt werden.

Auch die Verwendung eines Pd/BaSO₄-Katalysators zur Herstellung von Butendiol ist aus der DE-A 26 05 241 bekannt.

Aus M. Freifelder, "Practical Catalytic Hydrogenation", Wiley-Interscience, New York, 1971, S. 84 bis 126, ist eine Übersicht der technisch verwendeten Katalysatorsysteme für die Partialhydrierung von Dreifachbindungen zu olefinischen Doppelbindungen bekannt.

Alle genannten Verfahren haben den Nachteil, daß ein suspendierter Katalysator mit hohem Pd-Gehalt Verwendung findet. Der Katalysator muß nach erfolgter Hydrierung durch Absetzen und Filtrieren vom Reaktionsprodukt abgetrennt werden.

Es hat sich gezeigt, daß in technischem Maßstab die vollständige Abtrennung des pulverförmigen Katalysators nur mit sehr großem Aufwand möglich ist. Spuren von Katalysatorresten im Endprodukt verursachen aber bei der Weiterverarbeitung oder bei der sonstigen Verwendung der Alkene Schwierigkeiten. So hat es nicht an Versuchen gefehlt, einen Festbettkatalysator mit hoher Abriebfestigkeit für die partielle Hydrierung der Dreifachbindung in Alkinen in flüssiger Phase zu entwickeln.

Aus der EP-B1-04 12 415 ist ein Festbettkatalysator für die Hydrierung von 3,7-Dimethyl-oct-1-in-3-ol (Hydrodehydrolinalool) zu 3,7-Dimethyl-oct-1-en-3-ol (Hydrolinalool) bekannt, der als Aktivkomponente Palladium und als Inhibitor Metalle, wie Sn, Pb, Zn, Cd, Sb oder Bi enthält. Die in diesem Patent beschriebenen, mit Inhibitoren dotierten monolithischen Palladium-Festbettkatalysatoren ermöglichen es, die nachteilige Suspensionsfahrweise durch die technisch wesentlich vorteilhaftere Riesel- oder Sumpffahrweise am Festbettkatalysator zu ersetzen. Die recht hohe Abriebfestigkeit dieser Katalysatormonolithe ermöglicht eine sehr hohe Gas- und Flüssigkeitsbelastung. Leider hat sich bei der kontinuierlichen Durchführung des in diesem Patent beschriebenen Verfahrens an mit Wismut dotierten Palladium-Festbettkatalysatoren über längere Zeiträume gezeigt, daß die Selektivität der Hydrierung von Hydrodehydrolinalool zu Hydrolinalool langsam abnimmt, d.h. daß das Reaktionsprodukt ansteigende Mengen des vollständig hydrierten 3,7-Dimethyl-octan-3-ols enthält. Eine Untersuchung der austauschbedürftigen Katalysatoren ergab, daß der Katalysator seine Wismut-Dotierung verloren hatte. Versuche, diese Katalysatoren durch Tränken mit Wismut-Verbindungen und Reduzieren derselben zu regenerieren, waren nicht erfolgversprechend, da die Standzeiten der so regenerierten Katalysatoren nur sehr kurz waren.

Es war daher die Aufgabe der Erfindung, Festbettkatalysatoren für die Herstellung von Alkenen, vorzugsweise monosubstituierten Alkenen, durch Partialhydrierung der entsprechenden Alkine zu entwickeln, die die Vorteile der monolithischen Bi-dotierten Pd-Katalysatoren gemäß EP-B1-04 12 415 aufweisen, jedoch ihre Selektivität bei der kontinuierlichen Partialhydrierung von Alkinen zu Alkenen möglichst unbegrenzt behalten.

Es wurde nun überraschenderweise gefunden, daß man diese "verbrauchten" Katalysatoren, d.h. die monolithischen Palladium-Festbettkatalysatoren, die ihre Wismut-Dotierung verloren haben, mit guten Selektivitäten weiterverwenden kann, wenn man dem Hydrierwasserstoff sehr geringe Mengen CO zusetzt. Aber auch an Pd-Festbettkatalysatoren, die analog dem Verfahren gemäß EP-B1-04 12 415 hergestellt wurden, bei deren Herstellung man jedoch auf das Dotieren mit Inhibitoren und ggf. auch auf das anschließende Tempern von vornherein verzichtet hat, können Alkine mit guten Selektivitäten und sehr langen Standzeiten zu den entsprechenden Alkenen hydriert werden, wenn man dem Hydriergas geringe Mengen an CO zusetzt.

Gegenstand der Erfindung ist dementsprechend ein Verfahren zur Herstellung von Alkenen durch Partialhydrierung von Alkinen in der Flüssigphase an Palladiumkatalysatoren bei 20 bis 250°C und H₂-Partialdrucken von 0,3 bis 200 bar, daß dadurch gekennzeichnet ist, daß man
A. einen Festbett-Trägerkatalysator verwendet, der durch Tempern des Trägermaterials an der Luft, Abkühlen, Beschichten im Vakuum mit metallischem Palladium, Verformen und Verarbeiten zu monolithischen Katalysatorelement erhältlich ist und
B. daß man dem Hydriergas 10 bis 180 ppm, vorzugsweise 50 bis 150, insbesondere 60 bis 120 ppm CO zusetzt.

Besonders geeignet ist das Verfahren für die partielle Hydrierung von monosubstituierten Alkinen, wie 3,7-Dimethyl-oct-6-en-1-in-3-ol (Dehydrolinalool), 3,7-Dimethyl-oct-l-in-3-ol (Hydro-dehydrolinalool) oder 3-Methyl-1-butin-3-ol. Die partielle Hydrierung von monosubstituierten Alkinen ist bekanntlich wesentlich problematischer als die von disubstituierten Alkinen, wie Butin-1,4-diol, da sie während der Hydrierung weiterreagieren können. Jedoch ist auch die Partialhydrierung von disubstituierten Alkinen, wie Butin-1,4-diol mit dem erfindungsgemäßen Verfahren möglich.

Als Ausgangsprodukte für das erfindungsgemäße Verfahren seien beispielsweise genannt:
Monosubstituierte Alkine, wie Dehydrolinalool, Hydro-dehydrolinalool, 2-Methyl-l-butin-3-ol, 3-Methyl-1-butin-3-ol,
1-Ethinyl-2,6,6-trimethyl-cyclohexanol und 17-Ethinyl-androst-5-en-3β,17β-diol;
und disubstituierte Alkine, wie Butin-1,4-diol, 2-Butin-1-ol, 3-Hexin-1-ol, 2-Methyl-3-butin-2-ol, 2-Hydroxy-3-pentin, 2-Hydroxy-3-hexin, 6-Methyl-2-hydroxy-3-heptin, 4-Phenyl-2-hydroxy-3-butin, 3-Methyl-3-hydroxy-4-hexin, 4-Methyl-4-hydroxy-2-decin, 2,5-Dimethyl-3-hexin-2,5-diol, 1,1-Diethoxy-2-octin, 5-Diethylamino-2-hydroxy-3-pentin und Bis-(tetrahydro-2-pyranyloxy)-2-butin.

Als Katalysator-Trägermaterial können Gewebe aus anorganischen Materialien, wie Al₂O₃ und/oder SiO₂ oder aber Gewebe aus Drähten aus Kunststoffen, wie Polyamiden, Polyestern, Polypropylen, Polytetrafluorethylen u.a. verwendet werden. Besonders geeignet sind jedoch folien- bzw. gewebeartige Metallträger, d.h. Folien oder Drahtgewebe aus Metallen, wie Eisen, Federstahl, Kupfer, Messing, Aluminium, Neusilber, Nickel, Chromstahl oder Chromnickelstähle. Besonders bewährt haben sich Folien oder Gewebe aus Werkstoffen mit den Werkstoffnummern 1.4767, 1.4401 und 1.4301. Die Bezeichnung dieser Werkstoffe mit den genannten Werkstoffnummern folgt den Angaben der Werkstoffnummern in der "Stahleisenliste", herausgegeben vom Verein Deutscher Eisenhüttenleute; 8. Aufl., Seiten 87, 89 und 106; Verlag Stahleisen mbH, Düsseldorf 1990. Der Werkstoff der Werkstoffnummer 1.4767 ist auch unter dem Namen Kanthal bekannt. Diese metallischen Trägermaterialien werden durch oxidative Temperung, vorzugsweise an der Luft bei Temperaturen von 600 bis 1100°C, vorzugsweise 700 bis 1000°C vorbehandelt und anschließend im Vakuum mit Palladium beschichtet.

Das Beschichten mit Palladium erfolgt durch Aufdampfen oder durch Aufsputtern im Vakuum, d.h. bei Drücken von 10⁻² bis 10⁻¹⁰, vorzugsweise 10⁻³ bis 10⁻⁶ mbar. Als Vakuumaufdampftechniken kommen alle bekannten Beschichtungsverfahren in Betracht, insbesondere die thermische Verdampfung. Man kann aber auch eine sogenannte Flash-Verdampfung, eine Kathodenzerstäubung sowie Sputtern benutzen. Die thermische Verdampfung kann durch direkte oder indirekte Heizung erfolgen. Vorzugsweise benutzt man die Elektronenstrahlverdampfung. Bei dieser Methode wird das zu verdampfende Metall in einem Tiegel mittels eines Elektronenstrahls oberflächlich so stark erhitzt, daß es verdampft. Bezüglich näherer Einzelheiten über Methoden des Aufdampfens und Aufsputterns von Metallen im Vakuum verweisen wir auf das "Handbook of Thin Film Technology", Verlag Maissel und Gang, Mc Graw Hill, New York, 1970; "Thin Film Processes" von J.L. Vossen und W.Kern, Academic Press, N.Y. sowie auf EP-A- 01 98 435, worauf hiermit Bezug genommen wird.

Mit Vorteil beschichtet man das Trägermaterial mittels Vakuumverdampfungstechnik in "Dünnen Schichten", d.h. Belägen im Dickenbereich zwischen 0,2 nm und 100 nm, vorzugsweise 0,5 nm bis 20 nm.

Das so mit Palladium beschichtete Trägermaterial kann anschließend durch Tempern bei Temperaturen von 200 bis 800°C, vorzugsweise 300 bis 700°C für 0,5 bis 2 Stunden formiert werden. Je nach Art der Palladiumbeschichtung kann dieser Temperschritt nach dem Beschichten aber auch entfallen. Die so mit Pd beschichteten und ggf. nachgetemperten Katalysatorfolien, Katalysatornetze oder Katalysatorgewebe werden dann zweckmäßigerweise für den Einbau in den Hydrierreaktor in an sich bekannter Weise zu Monolithen bzw. zu Formkörpern, wie z.B. Sulzerpackungen, verformt. Dadurch lassen sich die gewünschten guten Strömungsverhältnisse im Reaktor herstellen.

Nach der Reduktion des Katalysators mit Wasserstoff bei Temperaturen von 20 bis 250°C, vorzugsweise 100 bis 200°C, die man vorteilhaft im Reaktor durchführt, ist der Katalysator für die erfindungsgemäße Partialhydrierung einsatzbereit.

Mit Vorteil gelingt das erfindungsgemäße Verfahren wenn man die Partialhydrierung kontinuierlich in einem Rohrreaktor in Riesel- oder Sumpffahrweise mit Produkt rückführung bei Querschnittsbelastungen von 20 bis 300 m³/m² h, vorzugsweise 100 bis 250 m³/m² h durchführt.

Weiterhin ist es von großem Vorteil, wenn man das Hydriergasgemisch aus Wasserstoff und CO im Kreis fährt und die Geschwindigkeit der Wasserstoffaufnahme - und damit die Selektivität - mittels der CO-Dosierung regelt.

Mit besonderem Vorteil gelingt die Partialhydrierung in technischem Maßstab, wenn man sie in der Sumpffahrweise durchführt, und das Kreisgas mittels des Flüssigkeitsstromes und einer geeigneten Vorrichtung, wie einem Flüssigkeits-Gas-Verdichter in feinster Verteilung in den Reaktor eindüst. Zusammen mit der Formgebung der Katalysatormonolithe und der beschriebenen Begasung des Reaktors erzielt man hohe Raumzeitausbeuten durch optimale Quervermischung und gute Hydrodynamik an der Katalysatorgrenzfläche. Die Partialhydrierungen werden, je nach Substanz, bei Temperaturen von 20 bis 250°C, vorzugsweise 60 bis 100°C durchgeführt.

Mit Vorteil wird die Partialhydrierung kontinuierlich in einem oder mehreren hintereinandergeschalteten Reaktoren durchgeführt. Der Wasserstoffpartialdruck liegt im Bereich von 0,3 bis 200 bar, vorzugsweise 0,5 bis 20 bar. Die Hydrierungen können mit und ohne Abgas gefahren werden. Die Geschwindigkeit der H₂-Aufnahme kann sehr einfach über die CO-Dosierung geregelt werden, was technisch einen großen Fortschritt bedeutet. Mit Hilfe des erfindungsgemäßen Verfahrens ist es möglich, zahlreiche als Riechstoffe oder Zwischenprodukte benötigte Alkene, insbesondere monosubstituierte Alkene, wie Linalool, Hydrolinalool oder 3-Methyl-1-buten-1-ol, auch im technischen Maßstab in einem kontinuierlichen Verfahren an relativ einfach herstellbaren, nur wenig Pd enthaltenden, und über lange Zeiträume stabilen Katalysatoren in guten Ausbeuten, guten Raum-Zeit-Ausbeuten und gleichbleibend guten Selektivitäten aus den entsprechenden Alkinen herzustellen.

Die Durchführung der Katalysatorherstellung und der erfindungsgemäßen Partialhydrierung im Vergleich mit derjenigen gemäß dem nächsten Stand der Technik wird mit den folgenden Beispielen näher erläutert.

### Beispiel 1 (Vergleichsbeispiel)

A. Katalysatorherstellung
   Ein glattes Edelstahlgewebe (Werkstoffnummer 1.4767) mit einer Maschenweite von 180µ und einem Drahtdurchmesser von 110µ wurde im Ultraschallbad gereinigt und anschließend 7 Stunden (h) bei 900°C an der Luft getempert. Ein 20 cm breiter Gewebestreifen wurde auf die in einer Ultrahochvakuum-Bedampfungsanlage installierte Wickelvorrichtung aufgespannt und anschließend kontinuierlich bei einem Druck von 10⁻⁶ mbar mit 2 nm Pd bedampft. Durch Rückspulen des Gewebes wurde die) ses in einem zweiten Bedampfungsschritt mit 0,7 nm Bi belegt. Nach dem Aufdampfen wurde das Katalysatorvorprodukt 30 Minuten (min) bei 600°C in einer Elektromuffel formiert. Dazu wurde der Temperofen in 40 min auf 600°C aufgeheizt, 30 min bei dieser Temperatur gehalten und dann abgeschaltet. Nach dem Erkalten wurde der Katalysator aus der Muffel entnommen und zu einem Monolithen verformt. Hierzu wurden 41,5 cm glattes Gewebe mit einer Zahnradwalze wellenartig verformt und mit 38 cm glattem Gewebe zusammengelegt und aufgerollt. Man erhielt so einen Monolithkatalysator mit einem Volumen von 76 cm³.
B. Selektive Hydrierung von 2-Dehydrolinalool (2-DHL) zu 2-Linalool (2 LIN) in Abwesenheit von CO.
   Es wurde 1 m³ des gemäß Beispiel 1A hergestellten Pd/Bi-Katalysators in Form von Metallmonolithen mit 600 mm Durchmesser und 200 mm Höhe in einen Rohrreaktor eingefüllt. 2-DHL wurde in Sumpffahrweise unter Rückführung mit einer Querschnittsbelastung von 200 m³/m² · h über den Katalysator geleitet. Gleichzeitig mit dem Flüssigkeitsstrom wurde Wasserstoff bei einem Partialdruck von 2 bar im Kreis gefahren. Bei einer Temperatur von 90°C erhielt man eine Raumzeitausbeute von 0,37L/L_{Kat} · h bei 100%igem Umsatz und einer Überhydrierung von nur 1,35 %.
   Es zeigte sich jedoch, daß die Überhydrierung im Laufe der Zeit größer wurde. Nach 46 Tagen hatte sie bereits einen Wert von 4,98 % und nach 131 Tagen von 6,37 %. Die Untersuchung des Katalysators ergab, daß unter diesen Reaktionsbedingungen das als Inhibitor verwendete Bi vollständig aus dem Katalysator ausgetragen worden war und dieser dadurch seine Selektivität verloren hatte.

### Beispiel 2

A. Katalysatorherstellung
   Das gleiche glatte Edelstahlgewebe wie in Beispiel 1A wurde im Ultraschallbad gereinigt und anschließend 7 h bei 900°C in der Luft getempert. Ein 20 cm breiter Gewebestreifen wurde auf die in einer UHV UHV-Bedampfungsanlage installierte Wickelvorrichtung aufgespannt und anschließend kontinuierlich bei einem Druck von 10⁻⁶ mbar mit 2 nm Pd bedampft. Anschließend wurde der Katalysator wie in Beispiel 1A beschrieben zu einem Monolithen verformt.
B. Selektive Hydrierung von 2-DHL zu 2-LIN an einem Pd-Katalysator in Gegenwart von CO.

Es wurde 1 m³ des gemäß Beispiel 2A hergestellten Pd-Katalysators in Form von Metallmonolithen mit 600 mm Durchmesser und 200 mm Höhe in den gleichen Rohrreaktor wie in Beispiel 1B eingefüllt. 2-DHL wurde analog Beispiel 1B in Sumpffahrweise unter Rückführung mit einer Querschnittsbelastung von 200 m³/m² · h über den Katalysator geleitet. Gleichzeitig mit dem Flüssigkeitsstrom wurde Wasserstoff, dem 70 ppm CO zugesetzt waren, bei einem Partialdruck von 1,5 bar im Kreis gefahren.

Die Menge an CO im Kreisgas wurde konstant gehalten. Bei 90°C, einem H₂-Partialdruck von 1,5 bar und 70 ppm CO im Kreisgas erhält man bei einer Raumzeitausbeute von 0,31 L/Lₖₐₜ · h bei einem Umsatz von 100 % eine Überhydrierung von 1,2 %. Nach einer Laufzeit von 100 Tagen betrug die Überhydrierung bei 100%igem Umsatz konstant 1,2 %. Eine Alterung des Katalysators wurde nicht mehr beobachtet.

## Patentansprüche

1. Verfahren zur Herstellung von Alkenen durch Partialhydrierung von Alkinen in der Flüssigphase an Palladiumkatalysatoren bei Temperaturen von 20-250°C und Wasserstoffpartialdrucken von 0,3 bis 200 bar, dadurch gekennzeichnet, daß man
A. einen Festbett-Trägerkatalysator verwendet, der durch Tempern des Trägermaterials an der Luft, Abkühlen, Beschichten im Vakuum mit metallischen Palladium, Verformen und Verarbeiten zu monolithischen Katalysatorelementen erhältlich ist, und
B. daß man dem Hydrierwasserstoff 10 bis 180 ppm Kohlenmonoxid zusetzt.

2. Verfahren gemäß Anspruch 1, dadurch gekennzeichnet, daß man einen Trägerkatalysator aus metallischem Trägermaterial in Form eines Metallgewebes oder einer Metallfolie verwendet.

3. Verfahren gemäß Anspruch 1, dadurch gekennzeichnet, daß man es zur Herstellung monosubstituierter Alkene aus den entsprechenden Alkinen verwendet.

4. Verfahren gemäß Anspruch 3, dadurch gekennzeichnet, daß man es zur Herstellung von 3,7-Dimethyl-oct-1,6-dien-3-ol, 3,7-Dimethyl-oct-1-en-3-ol oder 3-Methyl-1-buten-3-ol aus den entsprechenden Alkinen verwendet.

5. Verfahren gemäß den Ansprüchen 1 bis 4, dadurch gekennzeichnet, daß man die Partialhydrierung in einem Rohrreaktor in Riesel- oder Sumpffahrweise mit Produktrückführung bei Querschnittsbelastungen von 20 bis 300 m³/m².h durchführt.

6. Verfahren gemäß Anspruch 5, dadurch gekennzeichnet, daß man das Hydriergasgemisch aus Wasserstoff und Kohlenmonoxid im Kreis fährt und die Wasserstoffaufnahme -und damit die Selektivität- mittels der Kohlenmonoxid-Dosierung regelt.

7. Verfahren gemäß Anspruch 5, dadurch gekennzeichnet, daß man die Partialhydrierung in der Sumpffahrweise durchführt und das Kreisgas mittels einer geeigneten Vorrichtung in feinster Verteilung in den Reaktor eindüst.

8. Verfahren gemäß Anspruch 1, dadurch gekennzeichnet, daß man die Partialhydrierung bei Temperaturen von 60 bis 150°C durchführt.

9. Verfahren gemäß Anspruch 1, dadurch gekennzeichnet, daß man die Partialhydrierung bei einem Wasserstoffpartialdruck von 0,5 bis 20 bar durchführt.

10. Verfahren gemäß Anspruch 1, dadurch gekennzeichnet, daß man die Partialhydrierung kontinuierlich in einem oder mehreren hintereinandergeschalteten Reaktoren durchführt.

11. Verfahren gemäß Anspruch 1, dadurch gekennzeichnet, daß man einen Festbett-Trägerkatalysator verwendet, der dadurch erhältlich ist, daß er nach dem Beschichten des Trägermaterials mit Palladium zur Formierung nachgetempert wird.

## Claims

1. A process for the preparation of alkenes by partial hydrogenation of alkynes in the liquid phase over palladium catalysts at 20-250°C and hydrogen partial pressures of from 0.3 to 200 bar, which comprises
A. using a fixed-bed supported catalyst which is obtainable by heating the carrier in the air, cooling, coating under reduced pressure with metallic palladium, molding and processing to monolithic catalyst elements and
B. adding from 10 to 180 ppm of carbon monoxide to the hydrogen used for the hydrogenation.

2. A process as claimed in claim 1, wherein a supported catalyst comprising a metallic carrier in the form of a woven metal fabric or a metal foil is used.

3. A process as claimed in claim 1, which is used for the preparation of monosubstituted alkenes from the corresponding alkynes.

4. A process as claimed in claim 3, which is used for the preparation of 3,7-dimethyloct-1,6-dien-3-ol, 3,7-dimethyloct-1-en-3-ol or 3-methylbut-1-en-3-ol from the corresponding alkynes.

5. A process as claimed in any of claims 1 to 4, wherein the partial hydrogenation is carried out in a tube reactor by the trickle-bed or liquid phase procedure with product recycling with cross-sectional loadings of from 20 to 300 m³/m²·h.

6. A process as claimed in claim 5, wherein the hydrogenation gas mixture comprising hydrogen and carbon monoxide is circulated and the hydrogen absorption, and hence the selectivity, are regulated by means of the carbon monoxide metering.

7. A process as claimed in claim 5, wherein the partial hydrogenation is carried out by the liquid phase procedure and the cycle gas is sprayed in very fine distribution into the reactor by means of a suitable apparatus.

8. A process as claimed in claim 1, wherein the partial hydrogenation is carried out at from 60 to 150°C.

9. A process as claimed in claim 1, wherein the partial hydrogenation is carried out at a hydrogen partial pressure of from 0.5 to 20 bar.

10. A process as claimed in claim 1, wherein the partial hydrogenation is carried out continuously in one or more reactors connected in series.

11. A process as claimed in claim 1, wherein a fixed-bed supported catalyst which is obtainable by heating it for forming after coating of the carrier with palladium is used.

## Revendications

1. Procédé pour préparer des alcènes par hydrogénation partielle d'alcynes en phase liquide sur des catalyseurs au palladium à des températures de 20 à 250°C et des pressions partielles d'hydrogène de 0,3 à 200 bar, caractérisé par le fait que
**A.** on utilise un catalyseur sur support en lit fixe, obtenu par chauffage de la matière de support à l'air, refroidissement, revêtement par du palladium métallique sous vide, formage et conversion en éléments catalytiques monolithiques, et
**B.** on ajoute de 10 à 180 ppm de monoxyde de carbone à l'hydrogène prévu pour l'hydrogénation.

2. Procédé selon la revendication 1, caractérisé par le fait que l'on utilise un catalyseur sur support consistant en une matière de support métallique à l'état de toile métallique ou de feuille métallique.

3. Procédé selon la revendication 1, caractérisé par le fait que, pour préparer des alcènes monosubstitués, on part des alcynes correspondants.

4. Procédé selon la revendication 3, caractérisé par le fait qu'on l'exploite pour la préparation du 3,7-diméthyl-octa-1,6-diène-3-ol, du 3,7-diméthyl-octa-1-ène-3-ol ou du 3-méthyl-1-butène-3-ol à partir des alcynes correspondants.

5. Procédé selon les revendications 1 à 4, caractérisé par le fait que l'on réalise l'hydrogénation partielle dans un réacteur tubulaire, en phase ruisselante ou de culot, avec recyclage du produit, avec des charges moyennes de 20 à 300 m³/m².h.

6. Procédé selon la revendication 5, caractérisé par le fait que l'on fait circuler le mélange hydrogénant consistant en hydrogène et monoxyde de carbone et on règle l'absorption de l'hydrogène - et par conséquent la sélectivité - par le dosage de monoxyde de carbone.

7. Procédé selon la revendication 5, caractérisé par le fait que l'on réalise l'hydrogénation partielle en phase de culot et on injecte le gaz en circulation dans le réacteur à l'état de division aussi fine que possible à l'aide d'un dispositif approprié.

8. Procédé selon la revendication 1, caractérisé par le fait que l'on réalise l'hydrogénation partielle à des températures de 60 à 150°C.

9. Procédé selon la revendication 1, caractérisé par le fait que l'on réalise l'hydrogénation partielle sous une pression partielle d'hydrogène de 0,5 à 20 bar.

10. Procédé selon la revendication 1, caractérisé par le fait que l'on réalise l'hydrogénation partielle en continu dans un ou plusieurs réacteurs disposés en série.

11. Procédé selon la revendication 1, caractérisé par le fait que l'on utilise un catalyseur sur support en lit fixe obtenu par une nouvelle cuisson pour façonnage après revêtement de la matière de support par le palladium.
